Europäisches Patentamt

European Patent Office

Office européen des brevets

(11) Veröffentlichungsnummer: **0 406 963 B1**

(12) **EUROPÄISCHE PATENTSCHRIFT**

(45) Veröffentlichungstag der Patentschrift: **20.12.95**

(51) Int. Cl.⁶: **A61B 5/04**

(21) Anmeldenummer: **90201753.2**

(22) Anmeldetag: **02.07.90**

(54) **Verfahren zur Rekonstruktion der räumlichen Stromverteilung in einem biologischen Objekt und Anordnung zur Durchführung des Verfahrens**

(30) Priorität: **06.07.89 DE 3922150**

(43) Veröffentlichungstag der Anmeldung:
**09.01.91 Patentblatt 91/02**

(45) Bekanntmachung des Hinweises auf die Patenterteilung:
**20.12.95 Patentblatt 95/51**

(84) Benannte Vertragsstaaten:
**DE FR GB**

(56) Entgegenhaltungen:
**DE-A- 3 725 532**

**IEEE TRANSACTIONS ON MAGNETICS. vol. 22, no. 5, September 1986, NEW YORK US Seiten 874 - 876; S. Ueno et al.: "The MEG topography and the source model of abnormal neural activities associated with brain lesions"**

**IEEE TRANSACTIONS ON MAGNETICS. vol. 19, no. 3, Mai 1983, NEW YORK US Seiten 835 - 844; S.J. Williamson et al.: "Application of SQUID sensors to the investigation of neural activity in the human brain"**

(73) Patentinhaber: **Philips Patentverwaltung GmbH**
**Röntgenstrasse 24**
**D-22335 Hamburg (DE)**

(84) Benannte Vertragsstaaten:
**DE**

(73) Patentinhaber: **Philips Electronics N.V.**
**Groenewoudseweg 1**
**NL-5621 BA Eindhoven (NL)**

(84) Benannte Vertragsstaaten:
**FR GB**

(72) Erfinder: **Dössel, Olaf Helmut, Dr.**
**Grosse Twiete 3b**
**D-2081 Tangstedt (DE)**
Erfinder: **Kullmann, Walter Heinrich, Dr.**
**Bernburger Weg 13**
**D-2000 Hamburg 61 (DE)**

(74) Vertreter: **Hartmann, Heinrich, Dipl.-Ing. et al**
**Philips Patentverwaltung GmbH,**
**Röntgenstrasse 24**
**D-22335 Hamburg (DE)**

"SQUID based measuring technique"
M.Hoke , 1988-VCH-Verlag

"Bioelectric Current Image Reconstruction" ,
W.J.Dallas 1987- Medical Imaging

ELECTRQ MEDICA (Siemens). vol. 57, no.1,
1989, Erlangen DE Seiten 2-7; F. Gudden et
al.: "Ein Vielkanalsystem zur Biomagnetischen Diagnostik in Neurologie und Kardiologie : Prinzip, Methode und erste Ergebnisse"

**Beschreibung**

Verfahren zur Rekonstruktion der räumlichen Stromverteilung in einem biologischen Objekt und Anordnung zur Durchführung des Verfahrens.

Die Erfindung betrifft ein Verfahren zur Rekonstruktion der räumlichen Stromverteilung in einem biologischen Objekt, wobei zumindest eine Komponente des durch die Stromquellen hervorgerufenen Magnetfeldes an einer Mehrzahl von Punkten außerhalb des Objektes gemessen wird, wonach aus den Meßwerten die Stromverteilung an den innerhalb des Objektes befindlichen Volumenelementen rekonstruiert wird, sowie eine Anordnung zur Durchführung des Verfahrens. Eine solche Anordnung und ein solches Verfahren sind aus dem Aufsatz "SQUIDs und Bilder neuronaler Ströme" von O. Dössel und W. Kullmann (Phys. Bl.44 (1988) Nr. 11, Seiten 423-425, bekannt.

Bei der Rekonstruktion müssen dabei aus den außerhalb des zu untersuchenden Objektbereichs gemessenen magnetischen Feldern die Stromdichten in den einzelnen Volumenelementen, aus denen sich der dreidimensionale Objektbereich zusammensetzt, nach Größe und Richtung berechnet werden. Es läßt sich zeigen, daß dieses sogenannte inverse dreidimensionale Problem nicht eindeutig lösbar ist. Bei dem bekannten Verfahren werden daher Rekonstruktionsalgorithmen benutzt, die eine näherungsweise Berechnung der Stromverteilung gestatten. Die auf diese Weise ermittelte Verteilung weicht von der tatsächlichen Verteilung jedoch ab.

Es ist Aufgabe der vorliegenden Erfindung, ein Verfahren der eingangs genannten Art so auszugestalten, daß diese Abweichungen verringert werden. Diese Aufgabe wird erfindungsgemäß dadurch gelöst, daß in einer die morphologische Struktur des Objektes enthaltenden Darstellung die anatomischen Flächen vorgegeben werden, auf denen sich die Stromquellen voraussichtlich befinden und daß die Rekonstruktion auf die Volumenelemente beschränkt wird, die sich auf diesen Flächen befinden.

Die Erfindung basiert auf der Erkenntnis, daß bei vielen Aktivitäten des Gehirns bekannt ist, daß die neuronalen Stromquellen auf bestimmten Flächen angeordnet sind. So können Tumore aufgrund ihres Platzbedarfs epileptische Anfälle auslösen. Das Innere des Tumors ist elektrisch inaktiv, der epileptische Fokus (Stromquelle) befindet sich irgendwo auf der Peripherie des Tumors. Bei einigen Formen der fokalen Epilepsie, die nicht auf einen Tumor zurückzuführen sind, kommt es ebenfalls zu einem morphologisch veränderten Gebiet, auf dessen Peripherie sich der gesuchte Fokus mit großer Wahrscheinlichkeit befindet. Schließlich ist bekannt, daß evozierte Felder, die nach einer Stimulation der Sinnesorgane am Kopf gemessen werden können, ihren Ursprung in einem räumlich abgrenzbaren Bereich der gefalteten Hirnrinde haben.

Dementsprechend erfolgt die Rekonstruktion der Stromquellendichte nur für die vorgegebene (zweidimensionale) Fläche und nicht für einen dreidimensionalen Bereich in dem zu untersuchenden Objekt. Infolgedessen ergibt sich das zweidimensionale inverse Problem, das im Prinzip eindeutig lösbar ist. Die Rekonstruktion wird daher in der Praxis nur noch dadurch beeinträchtigt, daß die Messung des Magnetfeldes einer endlichen Zahl von Punkten erfolgt ist und daß mit einer endlichen Genauigkeit gemessen wird.

Es sei an dieser Stelle erwähnt, daß es aus der Veröffentlichung von J.W.H. Meijs et al., "The EEG and MEG, using a model of eccentric spheres to describe the head", IEEE Trans. Biomed. Eng., vol. BME-34, pp. 913-920, 1987, bereits bekannt ist, aus Kernresonanztomogrammen bzw. aus Computertomogrammen Informationen über die individuelle Kopf- bzw. Gehirngeometrie zu entnehmen und damit die Lage eines einzigen Stromdipols genauer zu ermitteln. Bei diesem bekannten Verfahren geht es darum, aus den an verschiedenen Meßpunkten ermittelten Werten der magnetischen Flußdichte die Lage eines einzigen tangentialen, punktförmigen Stromdipols in einem Volumenleiter zu bestimmen, und zwar derart, daß das gemessene Magnetfeld möglichst genau mit dem Magnetfeld übereinstimmt, das gemessen werden würde, wenn an der betreffenden Stelle der Stromdipol vorhanden wäre. Dabei werden Kernspintomogramme oder Computertomogramme verwendet, die die Morphologie des Kopfes zeigen, und in denen Gebiete mit ähnlicher elektrischer Leitfähigkeit markiert werden, so daß eine genauere Modellierung der Volumenströme im Gehirn möglich ist.

Eine Anordnung zur Durchführung des erfindungsgemäßen Verfahrens ist gekennzeichnet durch eine Anordnung zur Durchführung des Verfahrens mit einer Meßanordnung zur Bestimmung der magnetischen Flußdichte außerhalb des Objektes, einem Speicher zur Speicherung der dabei gewonnenen Meßwerte, einer Einheit zur Bestimmung derjenigen Volumenelemente, die sich auf vorgebbaren Flächen befinden und einer Rekonstruktionseinheit zur Bestimmung der Stromverteilung an diesen Volumenelementen aus den gespeicherten Werten.

Die Erfindung wird nachstehend anhand der Zeichnung näher erläutert. Es zeigen:

Fig. 1 eine Anordnung zum Messen der magnetischen Felder,

Fig. 2 ein Prinzipschaltbild einer Einheit zur Verarbeitung der gemessenen Signale.

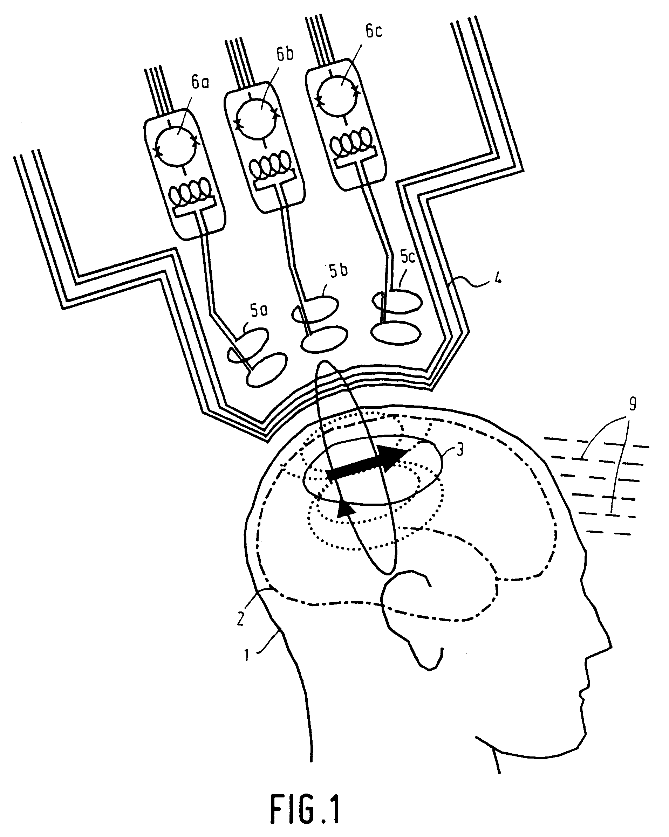

In Fig. 1 ist mit 1 der Schädel eines Patienten bezeichnet und mit 2 das darin durch strichpunktierte Linien angedeutete Gehirn. Mit 3 ist dabei die Oberfläche einer von ihrer Umgebung morphologisch abweichenden Struktur innerhalb des Gehirns bezeichnet, beispielsweise eines Tumors. Weiter ist angenommen, daß an der Grenzfläche des Tumors zum gesunden Gewebe, d.h. an seiner Oberfläche, ein durch elektrochemische Umsetzungsvorgänge entstandener eingeprägter Strom fließt, der durch einen dicken Pfeil dargestellt ist. Als Folge dieses eingeprägten Stroms fließen Volumenströme (gepunktet). Diese Volumenströme würden außerhalb des Schädels kein Magnetfeld erzeugen, falls der Kopf exakt als sphärischer Leiter betrachtet werden könnte.

Die außerhalb des Schädels gemessene Radialkomponente des Magnetfeldes hängt daher hauptsächlich von dem eingeprägten Strom ab, und zwar von dessen Tangentialkomponente. Durch Messung der Radialkomponente des Magnetfeldes an einer Mehrzahl von Punkten außerhalb des Schädels soll die Stromdichte der tangentialen Komponenten der eingeprägten Ströme als Funktion des Ortes bestimmt werden. Dabei können auch mehr als ein eingeprägter Strom lokalisiert werden, und es muß sich nicht um einen punktförmigen Stromdipol handeln.

Zur Messung der Radialkomponente der magnetischen Flußdichte befindet sich in einem oberhalb des Schädels des Patienten angeordneten Helium-Kryostaten 4 ein Meßsystem mit mehreren Meßkanälen, die jeweils ein supraleitendes Gradiometer (5a..5c) enthalten, das die durch den eingeprägten Strom erzeugte magnetische Flußdichte in jeweils einen SQUID (6a..6c) einkoppelt. Derartige Meßsysteme sind bekannt (DE-0S 37 35 668).

Im Ausführungsbeispiel ist ein Meßsystem mit nur drei Meßkanälen dargestellt, so daß die magnetische Flußdichte an nur drei Meßpunkten bestimmt werden kann. In der Praxis sollte aber das Magnetfeld an mehr als drei Meßpunkten gemessen werden, beispielsweise an 19 oder noch mehr. Daher ist ein Meßsystem mit einer höheren Anzahl von Kanälen erforderlich. Allerdings kann bei der Messung von evozierten Feldern, die nach einer Stimulation der Sinnesorgane gemessen werden können, auch ein Meßsystem mit nur einem Meßkanal benutzt werden, wenn dieses Meßsystem nacheinander in eine Reihe von definierten Positionen relativ zum Schädel gebracht wird. In jeder dieser Positionen wird dann der zeitliche Verlauf des Magnetfeldes gemessen, wobei der Bezugszeitpunkt jeweils der Zeitpunkt ist, an dem die Stimulation erfolgt. - Geeignete Meßverfahren zur gleichzeitigen oder aufeinanderfolgenden Messung des Magnetfeldes an verschiedenen Punkten sind in dem Artikel von M. Hoke "SQUID-Based Measuring Techniques", in "The art of Measurement", Ed. by B. Kramer, VCH Verlagsgesellschaft mbH, Weinheim, 1988, beschrieben.

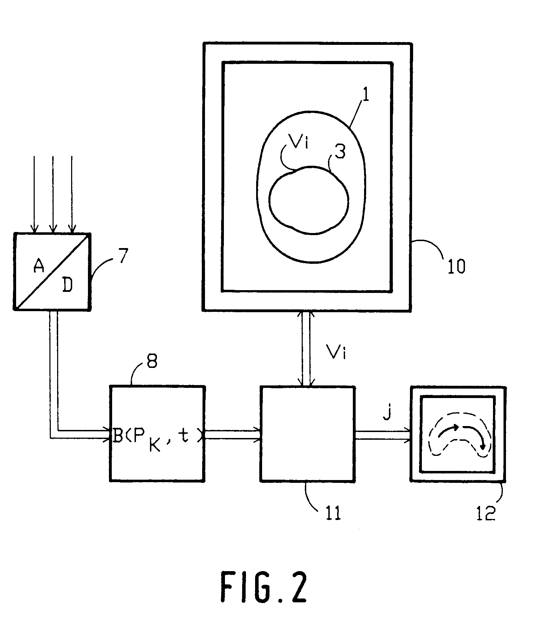

Die von den einzelnen Meßkanälen gelieferten analogen Meßsignale, die den zeitlichen Verlauf der Radialkomponente B der magnetischen Flußdichte darstellen, werden einer Analog-Digital-Wandleranordnung 7 zugeführt und jeweils in eine Folge digitaler Datenworte umgesetzt (Fig. 2). Diese Datenworte werden - gegebenenfalls nach einer mehrfachen Wiederholung der Messung und Mittelwertbildung, was bei der Messung der relativ schwachen evozierten Felder zur Verbesserung des Signal-Rausch-Verhältnisses erforderlich sein kann - in einem Speicher 8 gespeichert (die zur Steuerung der Meßkanäle und der Einheiten 7 und 8 erforderliche Steuereinheit ist in Fig. 2 der Einfachheit halber nicht näher dargestellt). Am Ende der Messung befindet sich also in dem Speicher ein Satz von Digitalwerten, die für jeden Meßpunkt $P_k$ - (mit k = 1..m, wobei m die Zahl der Meßpunkte ist), den zeitlichen Verlauf der Radialkomponente B der magnetischen Flußdichte darstellen.

Vor oder nach der Messung des Magnetfeldes wird die morphologische Struktur des Schädels bzw. des Gehirns ermittelt. Dies kann mittels eines Röntgen-Computertomographen erfolgen, der Computertomogramme von äquidistanten parallelen Schichten durch den Schädel des Patienten anfertigt, die in Fig. 1 durch eine Schar paralleler gestrichelter Linien 9 angedeutet sind. Stattdessen kann die Morphologie auch mittels einer Magnetresonanzuntersuchung ermittelt werden, wobei wiederum verschiedene (zweidimensionale) Magnetresonanztomogramme von parallelen Schichten angefertigt werden können, wobei aber auch von vornherein ein dreidimensionales Abbildungsverfahren benutzt werden kann. Die Morphologie kann aber auch auf andere Weise bestimmt werden, beispielsweise mit Hilfe der sogenannten Röntgen-Tomosynthese.

Wie bereits eingangs erläutert, läßt sich in vielen Fällen anhand der Morphologie des Schädels angeben, von welchen Flächen des Gehirns dessen elektrische Aktivitäten ausgehen. Dies gilt sowohl für evozierte Ströme, die durch Stimulation der Sinnesorgane auftreten, als auch für spontan, beispielsweise bei einem epileptischen Anfall auftretende Ströme. Die Vorgabe dieser Flächen erfolgt mit Hilfe einer Einheit 10, beispielsweise eines interaktiven Displays, mit dem der Benutzer die Flächen markiert, auf denen sich die Zentren der elektrischen Aktivität aller Voraussicht nach befinden. Dazu werden die Schichtbilder der Reihe nach auf der Einheit 10 wiedergegeben und die betreffenden Flächen vom Benutzer in geeigneter Weise, beispielsweise mit Hilfe eines sogenannten Lichtgriffels, markiert. Es ist aber auch möglich, die Flächen in einer automatischen Einheit 10 mit Hilfe eines geeigneten Konturfindungsalgorithmus zu bestimmen, der die Lage beispielsweise eines Tumors automatisch ermittelt. Die Einheit 10 liefert somit die Koordinaten derjenigen Volumenelemente (Voxel) $V_i$, die sich auf der gesuchten, im vorliegenden Beispiel mit 3 bezeichneten Fläche befinden. Diese Koordinaten sind auf geeignete Weise - beispielsweise durch Anbringen von Referenzmarken am Schädel 1 - denen des Magnetfeldmeßsystems angepaßt.

Eine Rekonstruktionseinheit 11 rekonstruiert die Dichte des eingeprägten Stroms an den einzelnen Voxeln $V_i$ (1..i..s, wobei s die Zahl der Voxel auf der markierten Fläche ist) aus den Meßwerten der Magnetflußdichte an den verschiedenen Bildpunkten zu jeweils dem gleichen Zeitpunkt. Dazu können die an sich bekannten Algorithmen benutzt werden.

Der Zusammenhang zwischen den Meßwerten der Magnetflußdichte B an den Meßpunkten $P_k$ und der Stromdichte J an den Voxeln $V_i$ läßt sich durch die Biot-Savart-Gleichung in Matrixform beschreiben: $B = A^*j + n$.

Dabei ist B eine Spaltenmatrix vom Typ (m,1), d.h. eine Matrix mit einer Spalte aus m-Elementen, die die magnetische Flußdichte an den m-Meßpunkten zum ausgewählten Zeitpunkt beschreiben. n ist eine Matrix gleichen Typs, die die Rauschkomponenten der magnetischen Flußdichte an den einzelnen Meßpunkten darstellt. j ist ebenfalls eine Spaltenmatrix mit 2s-Elementen, die jeweils die beiden (zueinander senkrechten) Tangential-Komponenten der Stromdichte an den s Voxeln auf der markierten Fläche 3 darstellen.

A ist die Biot-Savart-Matrix vom Typ (m,2s), deren Matrixelemente durch die geometrischen Beziehungen zwischen den Meßpunkten und den Voxeln definiert sind; die Matrixelemente sind also durch die geometrische Lage der ihnen zugeordneten Meßpunkte und Voxel eindeutig vorgegeben.

Eine Voraussetzung für die eindeutige Lösbarkeit der oben angegebenen Matrixgleichungen ist, daß die Matrix A den Rang n hat und daß n = 2s gilt. In diesem Fall kann die gesuchte Matrix für j unmittelbar durch Invertieren der Matrix A ermittelt werden. In den anderen Fällen - aber auch in dem angegebenen Fall - kann eine optimale Schätzung der Stromdichtematrix j mit Hilfe der sogenannten Moore-Penrose-Pseudoinversen ermittelt werden. Dieses Verfahren und seine Anwendung zur Rekonstruktion der Stromdichteverteilung ist bekannt u.a. aus dem Artikel von Dallas et al, "Bioelectric current image reconstruction from measurement of the generated magnetic fields", in "Medical Imaging", R.H. Schneider, S.J. Dwyer III, Editors, Proc. SPIE 767, pp. 2-10, 1987. Da im vorliegenden Fall die Rekonstruktion auf die von der Einheit 10 vorgegebenen Flächen bzw. Volumenelemente $V_i$ beschränkt ist, ist diese Rekonstruktion wesentlich genauer als bei den in der genannten Veröffentlichung beschriebenen Anwendungen, wo die Stromdichteverteilung an sämtlichen Voxeln aufgrund einer Schätzung rekonstruiert wird.

Ergebnis des Rekonstruktionsprozesses ist somit die Stromdichte j bzw. deren zueinander senkrechte tangentiale Komponenten an den über die Einheit 10 vorgegebenen Voxeln. Diese Verteilung läßt sich auf einem geeigneten Wiedergabegerät 12 wiedergeben, wobei der Darstellung zwecks einfacherer Orientierung noch ein Tomogramm überlagert sein kann, das die morphologischen Strukturen erkennen läßt, auf denen die ermittelten Ströme fließen.

**Patentansprüche**

1. Verfahren zur Rekonstruktion der räumlichen Stromverteilung in einem biologischen Objekt, wobei zumindest eine Komponente des durch die Stromquellen hervorgerufenen Magnetfeldes an einer Mehrzahl von Punkten außerhalb des Objektes gemessen wird, wonach aus den Meßwerten die Stromverteilung an den innerhalb des Objektes befindlichen Volumenelementen rekonstruiert wird,
dadurch gekennzeichnet, daß in einer die morphologische Struktur des Objektes enthaltenden Darstellung die anatomischen Flächen vorgegeben werden, auf denen sich die Stromquellen voraussichtlich befinden und daß die Rekonstruktion auf die Volumenelemente beschränkt wird, die sich auf diesen Flächen befinden.

2. Verfahren nach Anspruch 1,
dadurch gekennzeichnet, daß zur Ermittlung der vorzugebenden Flächen Röntgen-Computertomogramme von Schichten des Objektes angefertigt werden.

3. Verfahren nach Anspruch 1,
dadurch gekennzeichnet, daß zur Ermittlung der vorzugebenden Flächen ein dreidimensionaler Bereich des Objektes mittels eines Kernspin-Abbildungsverfahrens dargestellt wird.

4. Anordnung zur Durchführung des Verfahrens nach Anspruch 1 mit einer Meßanordnung (4,5,6) zur Bestimmung der magnetischen Flußdichte (B) außerhalb des Objektes, einem Speicher (8) zur Speicherung der dabei gewonnenen Meßwerte, einer Einheit (10) zur Bestimmung derjenigen Volumenelemente ($V_i$), die sich auf vorgebbaren Flächen (3) befinden und einer Rekonstruktionseinheit zur Bestimmung der Stromverteilung (j) an diesen Volumenelementen aus den gespeicherten Werten (8).

## Claims

1. A method of reconstructing the spatial current distribution in a biological object, at least one component of the magnetic field produced by the current sources being measured at a number of points outside the object, after which the current distribution at the volume elements situated within the object is reconstructed on the basis of the measuring values, characterized in that in a representation which contains the morphological structure of the object the anatomical surfaces on which the current sources are presumably present are specified, the reconstruction being limited to the volume elements situated on said surfaces.

2. A method as claimed in Claim 1, characterized in that X-ray computer tomograms of slices of the object are made in order to determine the surfaces to be specified.

3. A method as claimed in Claim 1, characterized in that a three-dimensional region of the object is reproduced by means of a magnetic resonance imaging method in order to determine the surfaces to be specified.

4. A device for performing the method claimed in Claim 1, comprising a measuring device (4,5,6) for determining the magnetic flux density (B) outside the object, a memory (8) for storing the measuring values thus acquired, a unit (10) for determining the volume elements ($V_i$) situated on surfaces (3) to be specified, and a reconstruction unit for determining the current distribution (j) at these volume elements from the values (8) stored.

## Revendications

1. Procédé de reconstruction de la distribution spatiale de courants dans un objet biologique, suivant lequel au moins une composante du champ magnétique produit par les sources de courant est mesurée en une pluralité de points à l'extérieur de l'objet, après quoi la distribution des courants sur les éléments de volume se trouvant à l'intérieur de l'objet est reconstruite à partir des valeurs de mesure, caractérisé en ce que les surfaces anatomiques sur lesquels les sources de courant se trouvent de façon prévisible sont définis au préalable dans une représentation contenant la structure morphologique de l'objet et que la reconstruction est limitée aux éléments de volume qui se trouvent sur ces surfaces.

2. Procédé suivant la revendication 1, caractérisé en ce que des tomographies computérisées par rayons X de couches de l'objet sont élaborées pour la détermination des plans définis au préalable.

3. Procédé suivant la revendication 1, caractérisé en ce qu'une région tridimensionnelle de l'objet est représentée par un procédé d'imagerie par spin nucléaire pour la détermination des plans définis au préalable.

4. Dispositif pour l'exécution du procédé suivant la revendication 1, caractérisé en ce qu'il comprend un dispositif de mesure (4, 5, 6) pour la détermination de la densité de flux magnétique (B) à l'extérieur de l'objet, une mémoire (8) pour stocker les valeurs de mesure ainsi acquises, une unité (10) pour la détermination de ceux des éléments de volume ($V_i$) qui se trouvent sur des plans qui peuvent être définis au préalable et une unité de reconstruction pour la détermination de la distribution de courant (j) sur ces éléments de volume à partir des valeurs en mémoire (8).

FIG.1

FIG.2